(19) **Europäisches Patentamt / European Patent Office / Office européen des brevets**

(11) **EP 3 778 911 A1**

(12) # EUROPEAN PATENT APPLICATION
published in accordance with Art. 153(4) EPC

(43) Date of publication:
**17.02.2021 Bulletin 2021/07**

(21) Application number: **19778327.7**

(22) Date of filing: **26.03.2019**

(51) Int Cl.:
**C12P 13/02** (2006.01)     **C07C 231/06** (2006.01)
**C07C 233/09** (2006.01)

(86) International application number:
**PCT/JP2019/013039**

(87) International publication number:
**WO 2019/189277 (03.10.2019 Gazette 2019/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.03.2018 JP 2018062126**

(71) Applicant: **Mitsui Chemicals, Inc.**
**Minato-ku**
**Tokyo 105-7122 (JP)**

(72) Inventors:
- **YAMAMOTO, Maki**
  **Sodegaura-shi, Chiba 299-0265 (JP)**
- **KOTAKI, Yasushi**
  **Sodegaura-shi, Chiba 299-0265 (JP)**
- **ISHIDA, Tsutomu**
  **Takaishi-shi, Osaka 592-8501 (JP)**

(74) Representative: **Mewburn Ellis LLP**
**Aurora Building**
**Counterslip**
**Bristol BS1 6BX (GB)**

(54) **METHOD FOR PRODUCING AMIDE COMPOUND**

(57)     Provided is a method of producing an amide compound, the method including: obtaining a reaction solution containing an amide compound by bringing a microbial cell containing nitrile hydratase, or a processed product of the microbial cell, into contact with a nitrile compound in an aqueous medium in a first reactor; and causing the obtained reaction solution containing an amide compound to react in a second reactor having a plug-flow region, in which the Reynolds number in the second reactor is controlled to from 5 to 1,000.

EP 3 778 911 A1

**Description**

Technical Field

The present disclosure relates to a method of producing an amide compound.

Background Art

The hydration method using a nitrile compound as a raw material is used as one of the major process for producing an amide compound in many cases. Particularly, it is known that acrylamide is produced from acrylonitrile as a raw material conventionally with a metallic copper catalyst, such as Raney copper, and in recent years, with a hydration catalyst, such as a microbial cell containing nitrile hydratase or a processed product of the microbial cell. Especially, a production method using nitrile hydratase is advantageous in that, for example, the reaction conditions are mild, the method yields a high-purity product, and the production process can be simplified.

Microbial cell catalysts have poor heat resistance and their catalytic activities are reduced (inactivated) without the removal of reaction heat; therefore, in the industrial use thereof, it is important to attain satisfactory productivity while inhibiting inactivation. For this purpose, as a method that efficiently utilizes a microbial cell catalyst, International Publication No. WO 2003/000914 discloses a method of continuously producing an amide compound in which the temperature of a reaction vessel on the downstream side is increased. Japanese Patent Application Laid-Open (JP-A) Nos. 2001-340091 and 2013-162746 each disclose a method in which a reactor having a plug-flow region is utilized. As the reactor having a plug-flow region, tube reactors with a double-tube configuration or a shell-and-tube configuration, as well as those provided with a porous plate or a packing material for the purpose of imparting plug-flow characteristics are exemplified.

For general reactions, a tube reactor is more advantageous than a tank reactor in that the reactor volume can be reduced. However, since an ordinary liquid phase reaction requires a long reaction time, the tube may be excessively long in a case in which a tube reactor is used. In addition, it is easier to maintain a uniform temperature in a tank reactor. Therefore, a tank reactor is employed in many cases. By utilizing the advantages of a tank reactor and a tube reactor to allow most of a reaction to proceed in the tank reactor and to perform a final phase of the reaction in the tube reactor, the number of reactors can be reduced and an optimum reaction system can be constructed.

Technical Problem

The amide compound production method disclosed in JP-ANo. 2001-340091, in which a tank reactor and a tube reactor having a plug-flow region are used in combination, is still not satisfactory in terms of an increase in the reaction conversion rate and, therefore, there is room for improvement. Moreover, there are not many findings that offer a guideline for designing a tube reactor for a liquid-phase reaction.

In view of the above, an object of the present disclosure is to provide a method of producing a nitrile compound with a high conversion rate by using a reactor having a flow region with plug-flow characteristics.

Solution to Problem

The present inventors conducted intensive research in order to solve the above-described problems. The present inventors focused on and researched the Reynolds number in a tube reactor used in a method of continuously producing a high-concentration aqueous amide compound solution by hydration of a nitrile compound at a high conversion rate, and discovered that the reaction conversion rate can be improved using a tube reactor producing a given range of Reynolds number.

That is, one embodiment of the present invention encompasses the followings.

<1> A method of producing an amide compound, the method including:

obtaining a reaction solution containing an amide compound by bringing a microbial cell containing nitrile hydratase, or a processed product of the microbial cell, into contact with a nitrile compound in an aqueous medium in a first reactor; and
causing the obtained reaction solution containing an amide compound to react in a second reactor having a plug-flow region,
a Reynolds number in the second reactor being controlled to from 5 to 1,000.

<2> The method of producing an amide compound according to <1>, wherein the Reynolds number in the second reactor is 10 or higher.

<3> The method of producing an amide compound according to <1> or <2>, wherein the Reynolds number in the second reactor is 100 or lower.

<4> The method of producing an amide compound according to any one of <1> to <3>, wherein the second reactor includes a tube reactor that has a tube diameter of 10 cm or more.

Advantageous Effects of Invention

According to one embodiment of the invention, there can be provided a method of producing a nitrile compound with a high conversion rate by using a reactor having a flow region with plug-flow characteristics.

DESCRIPTION OF EMBODIMENTS

Hereinbelow, embodiments of the invention are described. However, the invention is not limited thereto and may be modified as appropriate.

<Method of Producing Amide Compound>

The present disclosure relates to a method of producing an amide compound, the method including: obtaining a reaction solution containing an amide compound by bringing a microbial cell containing nitrile hydratase, or a processed product of the microbial cell, into contact with a nitrile compound in an aqueous medium in a first reactor; and causing the obtained reaction solution containing an amide compound to react in a second reactor having a plug-flow region, in which the Reynolds number (hereinafter, may be referred to as "Re number") in the second reactor is controlled to from 5 to 1,000.

(Nitrile Hydratase)

In the method of producing an amide compound according to the present disclosure, a microbial cell containing nitrile hydratase or a processed product of the microbial cell is used. The nitrile hydratase in the present disclosure refers to an enzyme that is capable of hydrating a nitrile compound to generate a corresponding amide compound. The microbe containing nitrile hydratase is not particularly limited as long as it produces nitrile hydratase capable of hydrating a nitrile compound to generate a corresponding amide compound, and maintains the nitrile hydratase activity in a 30%-by-mass aqueous acrylamide solution. Specifically, preferable examples of the microbe include those belonging to the genus *Nocardia,* the genus *Corynebacterium,* the genus *Bacillus,* the thermophilic genus *Bacillus,* the genus *Pseudomonas,* the genus *Micrococcus,* the genus *Rhodococcus* represented by the *rhodochrous* species, the genus *Acinetobacter,* the genus *Xanthobacter,* the genus *Streptomyces,* the genus *Rhizobium,* the genus *Klebsiella,* the genus *Enterobacter,* the genus *Erwinia,* the genus *Aeromonas,* the genus *Citrobacter,* the genus *Achromobacter,* the genus *Agrobacterium,* and the genus *Pseudonocardia* represented by the *thermophila* species.

The microbe in the present disclosure also encompasses a transformant obtained by allowing an arbitrary host to express the nitrile hydratase gene cloned from any of the above-exemplified microbes. One representative example of the arbitrary host is *Escherichia coli* used in the below-described Examples; however, the arbitrary host is not particularly limited thereto. Examples of the arbitrary host also include bacteria belonging to the genus *Bacillus* such as *Bacillus subtilis,* as well as other microbial strains of yeasts, actinomycetes and the like. Examples thereof include MT-10822 (this strain has been deposited with the National Institute of Bioscience and Human-Technology, Agency of Industrial Science and Technology, Ministry of International Trade and Industry, 1-1-3 Higashi, Tsukuba-shi, Ibaraki, Japan (currently, National Patent Microorganism Depositary, Biological Resource Center, National Institute of Technology and Evaluation, 2-5-8 Kazusakamatari, Kisarazu-shi, Chiba, Japan), under accession No. FERM BP-5785 on February 7, 1996, based on the Budapest Treaty on the International Recognition of the Deposit of Microorganisms for the Purposes of Patent Procedure). Further, the microbe in the present disclosure encompasses a transformant that expresses a mutant nitrile hydratase with further improved acrylamide resistance, acrylonitrile resistance, and temperature resistance by replacement, deletion, cancellation, or insertion of one or more constituent amino acids of the enzyme with other amino acids using recombinant DNA technology.

In the production of an amide compound using any of the above-described microbes, cells of the microbe, or processed cells of the microbe are usually used. These cells can be prepared by a general method that is known in the fields of molecular biology, biotechnology, and genetic engineering. For example, a method in which the microbe is inoculated into an ordinary liquid culture medium such as an LB medium or an M9 medium, and then the microbe is grown at an appropriate culture temperature (which is generally from 20°C to 50°C, but may be 50°C or higher in the case of a thermophile) and subsequently separated and recovered from the culture medium by centrifugation, may be employed.

The processed product of a microbe in the present disclosure encompasses, for example, an extract or a ground product of the microbial cells, a post-separation product obtained by separating and purifying a nitrile hydratase active fraction of the extract or ground product, and a fixation product obtained by fixing the microbial cells, or the extract, ground product, or post-separation product thereof, using an appropriate carrier. These materials each correspond to the processed product of the present disclosure as long as they have a nitrile hydratase activity. These materials may be used singly, or two or more kinds thereof in different forms may be used simultaneously or alternately.

(Nitrile Compound)

In the present disclosure, a nitrile compound is brought into contact with a microbial cell containing nitrile hydratase or a processed product of the microbial cell in an aqueous medium. The nitrile compound is fed into the aqueous medium contained in the first reactor from, for example, a nitrile compound feed line. In the present disclosure, the type of the nitrile compound is not particularly limited, and the nitrile compound is specifically one having from about 2 to 20 carbon atoms, and examples thereof include a wide range of nitriles, such as aliphatic nitriles and aromatic nitriles. Examples of the aliphatic nitriles include saturated or unsaturated nitriles having from 2 to 6 carbon atoms, for example, aliphatic saturated mononitriles, such as acetonitrile, propionitrile, butyronitrile, isobutyronitrile, valeronitrile, isovaleronitrile, and capronitrile; aliphatic saturated dinitriles, such as malononitrile, succinonitrile, and adiponitrile; and aliphatic unsaturated nitriles, such as acrylonitrile, methacrylonitrile, and crotononitrile. Examples of the aromatic nitriles include benzonitrile, o-, m- and p-chlorobenzonitrile, o-, m- and p-fluorobenzonitrile, o-, m- and p-nitrobenzonitrile, o-, m- and p-tolunitrile, and benzyl cyanide. Among these, for example, acrylonitrile, methacrylonitrile, and crotononitrile are preferable.

(Aqueous Medium)

In the present disclosure, a reaction that yields an amide compound is performed in an aqueous medium containing the nitrile compound. Examples of the aqueous medium used in the present disclosure include water, and an aqueous solution in which a buffer such as a phosphate, an inorganic salt such as a sulfate or a carbonate, an alkali metal hydroxide, an amide compound or the like is dissolved at an appropriate concentration. The water is not particularly limited, and purified water such as distilled water or ion exchanged water can be used. The water is fed to the first reactor from a raw material water feed pipe. Further, other feed line may be separately arranged in a case in which a component other than water is added.

(Step in First Reactor)

The present disclosure includes the step of bringing a microbial cell containing nitrile hydratase, or a processed product of the microbial cell, into contact with the nitrile compound in the aqueous medium in the first reactor (this step may be hereinafter referred to as "first reaction step"). In this first reaction step, a reaction solution containing an amide compound is obtained. The reaction system when the amide compound is obtained from the nitrile compound using the microbial cell containing nitrile hydratase or the processed product of the microbial cell may be carried out by two or more reactors. In the first reaction step, the microbial cell or the processed product of the microbial cell, the nitrile compound, and the aqueous medium are fed to the first reactor. The reaction system in this step is not particularly limited and, for example, it may be a suspended bed or a fixed bed. In general, a suspended bed in a tank reactor equipped with a stirrer is more preferably used because of the easiness of removing reaction heat. In this case, the microbial cell containing nitrile hydratase or the processed product of the microbial cell may be fed from a microbial cell catalyst tank.

In the present disclosure, the concentration of the nitrile compound fed to the first reactor is preferably not lower than the saturation concentration of the nitrile compound at the start of the reaction. An upper limit of the concentration is not particularly limited; however, when the nitrile compound is fed in a large excess, it is necessary to use not only a large amount of a catalyst and a reactor having an excessively large capacity for the completion of the reaction but also an excessively large heat exchanger or the like for the removal of heat, which leads to a large financial burden in terms of equipment cost. Therefore, the nitrile compound is preferably fed at such a concentration that, when the nitrile compound is entirely converted into a corresponding amide compound and this amide compound is acrylamide, the theoretical concentration of the resulting solution is in a range of from 40% by mass to 80% by mass, more specifically the amount of acrylonitrile is from 0.4 parts by weight to 1.5 parts by weight with respect to 1 part by weight of water.

(Step in Second Reactor)

The present disclosure includes causing the reaction solution containing an amide compound obtained in the first reaction step to react in a second reactor having a plug-flow region (this step may be hereinafter referred to as "second reaction step"). The second reactor used in the present disclosure is a reactor having a plug-flow region, which is generally referred to as "tube reactor" or "tubular reactor". The second reactor has a configuration in which a reaction proceeds while a reaction solution is moved with a piston flow property inside a tube having a pipe shape, and examples thereof include those having a double-tube configuration or a shell-and-tube configuration for removing reaction heat. A mode of feeding a liquid into the second reactor is not limited and may be, for example, feeding by an upward flow or a downward flow.

Further, a reactor provided with a porous plate or a packing material for the purpose of maintaining a uniform fluid state of the reaction solution in the reactor may also be used as the reactor having a flow region with plug-flow characteristics, as long as it can produce a flow region with plug-flow characteristics in the reaction solution depending on the conditions such as flow rate.

In the method of producing an amide compound according to the present disclosure, the Re number in the second reactor is controlled to from 5 to 1,000. The Re number, which is the Reynolds number of a reaction fluid and means a ratio between inertial force and viscous force, is a dimensionless number that serves as an index of fluidity and is represented by the following Formula:

$$Re = \rho u d / \mu$$

wherein, $\rho$ represents a fluid density [kg/m$^3$]; u represents an average flow rate in a tube [m/s]; d represents a tube diameter [m]; and $\mu$ represents a fluid viscosity [Pa·s].

The values of density and the viscosity of a fluid are inherent to the fluid while they are affected by temperature thereof. Therefore, the Re number is adjusted based on the average flow rate u and the tube diameter d, namely the flow rate and the reactor size.

The density can be measured by, for example, a hydrometer method, a pycnometer method, or a vibration densitometer method. The viscosity can be measured by, for example, a capillary viscometer method, a falling ball viscometer method, or a rotary viscometer method.

In the present disclosure, the Re number is calculated from the average flow rate in the tube, the diameter of the tube, and the density and the viscosity of the reaction solution containing an amide compound prior to its feed to the second reactor. The density and the viscosity of the reaction solution containing an amide compound are values measured at the temperature of the inside of the second reactor.

In a reactor having a flow region with plug-flow characteristics, there is a Peclet number (Pe number), which is a dimensionless number that serves as an index of plug flow characteristics. The Pe number, which means a ratio between convection velocity and diffusion velocity and is a dimensionless number that serves as an index of plug flow characteristics, is represented by the following Formula in the case of an empty tube:

$$Pe = u d / D$$

wherein, D represents a diffusion coefficient [m$^2$/s] and is determined by dividing a thermal conductivity $\lambda$ [W/m/K] by a heat capacity Cp [J/kg/K] and a density $\rho$; u represents an average flow rate in the tube [m/s]; and d represents a tube diameter [m].

The value of D is inherent to a given fluid, and the measurement thereof is not easy compared with the density and the viscosity of the fluid that are used for calculating the Re number. A high Pe number means superior plug flow characteristics. The Pe number is controlled by the average flow rate u and the tube diameter d similarly to the Re number, and an increase or decrease of the Pe number shows the same tendency as that of the Re number. In other words, when the fluid average flow rate u and the tube diameter d are adjusted such that the Re number is increased, the value of the Pe number tends to be higher. Meanwhile, when the fluid average flow rate u and the tube diameter d are adjusted such that the Re number is reduced, the value of the Pe number tends to be lower. Therefore, in the method of producing the amide compound according to the present disclosure, an appropriate range is defined for the Re number that can be measured more simply and is more practical than the Pe number.

The reaction rate per unit volume decreases as the fluid flow deviates from the plug-flow region. In order to ensure plug-flow characteristics, it is required that the fluid flows in a laminar flow and the effects of mixing and diffusion in the axial direction, namely the flow direction, should be negligible. For this reason, the Re number needs to be 5 or higher and in view of achieving a higher conversion rate, the Re number is preferably 10 or higher, more preferably 11 or higher.

In order to prevent the fluid flow from developing into a turbulent flow and inhibiting an excessive increase in the equipment-related burden due to an excessive increase in the length of a tubular reactor, the Re number needs to be 1,000 or lower, preferably 500 or lower, more preferably 300 or lower and in view of achieving a higher conversion rate, it is still more preferably 100 or lower.

The Re number can be controlled by adjusting the diameter and the length of the tube, in a case in which the flow rate (feed amount) of the reaction solution, namely the amount of amide compound production, is fixed. The reactor volume required for completing the reaction increases as the flow rate is increased; however, the length of the tube can be controlled not to be excessively large by increasing the number of tubes in a shell-and-tube configuration. While a smaller diameter (inner diameter) of the tube leads to superior temperature control, the number of required tubes may be increased and the size of the reactor may be increased. In addition, a highly viscous fluid can cause clogging of such a thin tube. Therefore, in view of operational safety and easiness of maintenance, the diameter of the tube is preferably 5 cm or more and in view of further reducing the size of the reactor, the diameter of the tube is more preferably 10 cm or more, still more preferably than 12 cm or more. An upper limit of the diameter of the tube is not particularly limited as long as it is set such that the Re number is from 5 to 1,000 in the relation to the fluid density $\rho$ and the average flow rate u in the tube. In the case of a reactor provided with a porous plate or a packing material to produce plug-flow characteristics, the diameter of the tube tends to be greater than in a reactor that does not include these materials. In the case of a reactor provided with a porous plate or a packing material, the diameter of the tube may be, for example, 300 cm or less. Meanwhile, in the case of a reactor that does not provided with these materials, the diameter of the tube may be, for example, 30 cm or less.

In the present disclosure, the first reactor may be connected with a reactor other than the second reactor. For example, a reactor having a configuration similar to that of the first reactor may be connected to the first reactor, and a reactor having a configuration similar to that of the second reactor may be connected to the second reactor. The first reactor and the second reactor may be connected via a connecting pipe, and in such a case, another reactor may be arranged between the first reactor and the second reactor.

The amount of a catalyst to be used in the first reactor and the second reactor can be changed in accordance with the reaction conditions as well as the type and the form of the catalyst, and it may be, for example, from 10 ppm by mass to 50,000 ppm by mass, preferably from 50 ppm by mass to 30,000 ppm by mass, in the reaction solution based on the weight of dry microbial cells.

While amidation reaction is usually performed at or near normal pressure, it may be performed under increased pressure so as to improve the solubility of the nitrile compound in an aqueous medium. The reaction temperature is not particularly limited as long as it is not lower than the freezing point of the aqueous medium and, usually, the amidation reaction is performed at a temperature in a range of preferably from 0 to 50°C, more preferably from 10 to 40°C. The reaction can also be performed in a slurry state in which the resulting product is crystallized in the reaction solution. Moreover, the pH of the reaction solution during the amidation reaction is not particularly limited as long as the nitrile hydratase activity is maintained, and it is preferably in a range of from 6 to 10, more preferably in a range of from 7 to 9.

In the present disclosure, a step other than the above-described steps may or may not be performed. Examples of the step other than the above-described steps include a step of purifying the amide compound by bringing the amide compound produced using the microbial cell containing a nitrile hydratase catalyst into contact with an activated carbon.

Examples of the activated carbon generally include those obtained from coal, wood, or coconut shell as a raw material. The activated carbon is not particularly limited as long as it has an adsorption capacity, and any such activated carbon can be used. However, in a case in which the amide compound to be treated has an unsaturated bond in particular, taking into consideration the storage stability and the high polymerizability of the amide compound, it is preferable to use an activated carbon having a low metal content, and it is more preferable to use an activated carbon obtained from a woody raw material or coconut shell.

Regarding the amount of the activated carbon to be used in the purification treatment of the amide compound, it is difficult to attain a sufficient purification effect in a case in which the amount of the activated carbon is excessively small, while it is not economical in a case in which the amount of the activated carbon is excessively large. Therefore, the activated carbon is used in an amount of usually in a range of from 0.01% by mass to 20% by mass, preferably in a range of from 0.05% by mass to 10% by mass, with respect to the amount of the solution containing an amide compound. Specifically, in a case in which a powder-form activated carbon is used, the activated carbon may be directly added to the solution containing an amide compound, or may be once dispersed in a medium such as water, and then the resulting slurry-form activated carbon may be added or fed to the solution containing an amide compound.

Subsequently, in the present disclosure, a purified liquid of the solution containing an amide compound may be obtained by separating the activated carbon from the solution containing an amide compound subjected to the above-described contact treatment. The method of separating the activated carbon is not particularly limited as long as it employs a generally used solid-liquid separation apparatus, and examples thereof include a pressure filtration apparatus, a vacuum filtration apparatus, and a centrifuge. The solid-liquid separation apparatus may be of a batch type or a continuous type. In the present disclosure, it is also possible to obtain a further purified amide compound by employing a method of cooling the solution containing an amide compound from which the activated carbon has been separated and thereby allowing the amide compound of interest to crystallize out of the solution.

In the present disclosure, a pH modifier may be used for adjusting the pH of the solution containing an amide compound to be in a range suitable for maintaining a favorable purification efficiency in the purification treatment.

In a case in which the pH suitable for the purification treatment is lower than 7, an acid can be used as the pH modifier.

The acid used as the pH modifier may be an inorganic acid or an organic acid. Examples of the inorganic acid include hydrohalic acids, such as hydrogen chloride, hydrogen bromide, and hydrogen iodide; halogenated oxoacids, such as hypochlorous acid, chlorous acid, chloric acid, perchloric acid, hypobromous acid, bromous acid, bromic acid, perbromic acid, hypoiodic acid, hypoiodous acid, iodic acid, and periodic acid; sulfuric acid, nitric acid, phosphoric acid, and boric acid. Examples of the organic acid include carboxylic acids, such as formic acid, acetic acid, propionic acid, acrylic acid, methacrylic acid, crotonic acid, oxalic acid, malonic acid, fumaric acid, maleic acid, citric acid, lactic acid, and benzoic acid; and sulfonic acids, such as methanesulfonic acid, ethanesulfonic acid, benzenesulfonic acid, and *p*-toluenesulfonic acid. The acid used as the pH modifier may be in any state of gas, solid, and liquid. Taking into consideration the easiness of feeding the acid into a purification tank, it is preferable to use an acid in a liquid state, and it is more preferable to use an acid in a gas or solid state in the form of an aqueous solution. Taking into consideration the controllability in pH adjustment of the purification tank, it is also more preferable to use an acid in a liquid state in the form of an aqueous solution. The concentration of the acid used in the form of an aqueous solution is not particularly limited. Since it is difficult to adjust the pH in a case in which a high-concentration aqueous solution is used, the concentration of the acid is preferably from 0.1% by mass to 99% by mass, more preferably from 1% by mass to 90% by mass, still more preferably 1% by mass to 50% by mass.

In a case in which the pH suitable for the purification treatment is higher than 7, a base can be used as the pH modifier.

The base used as the pH modifier may be an inorganic base or an organic base. Examples of the inorganic base include alkali metal hydroxides, such as lithium hydroxide, sodium hydroxide, and potassium hydroxide; alkaline earth metal hydroxides, such as magnesium hydroxide and calcium hydroxide; alkali metal carbonates, such as lithium carbonate, sodium carbonate, and potassium carbonate; alkali metal bicarbonates, such as lithium bicarbonate, sodium bicarbonate, and potassium bicarbonate; and ammonia. Examples of the organic base include trimethylamine, triethylamine, aniline, and pyridine. The base used as the pH modifier may be in any state of gas, solid, and liquid. Taking into consideration the easiness of feeding the base into a purification tank, it is preferable to use a base in a liquid state, and it is more preferable to use a base in a gas or solid state in the form of an aqueous solution. Taking into consideration the controllability in pH adjustment of the purification tank, it is also more preferable to use a base in a liquid state in the form of an aqueous solution. The concentration of the base used in the form of an aqueous solution is not particularly limited. Since it is difficult to adjust the pH in a case in which a high-concentration aqueous solution is used, the concentration of the base is preferably from 0.1% by mass to 99% by mass, more preferably from 1% by mass to 90% by mass, still more preferably 1% by mass to 50% by mass.

EXAMPLES

Hereinbelow, the present disclosure is described in more detail by referring to the examples, but the present disclosure is not limited at all to these examples. In the below-described examples, "%" means "% by mass" unless otherwise specified.

[Example 1]

[Preparation of Microbial Cell Containing Nitrile Hydratase]

A microbial cell of clone No. 3 was obtained in accordance with the method described in Example 1 of JP-ANo. 2001-340091, and the microbial cell was cultured in the same manner as in Example 1 of JP-ANo. 2001-340091, thereby obtaining a wet microbial cell containing nitrile hydratase.

[Production of Acrylamide]

An SUS tank reactor (capacity: 1 $m^3$) having a jacket heat exchanger, equipped with a stirrer, and having a tank inner diameter of 1 m and a straight body-portion length of 1.5 m, and an SUS multitubular cylindrical reactor having a capacity of 0.4 $m^3$ were prepared as a first reactor and a second reactor, respectively. The second reactor was configured to have 15 tubes of 89.1 mm in outer diameter, 80.7 mm in inner diameter, and 5 m in tube length. The second reactor was vertically set, and a reaction solution was fed from a lower part of each tube, and cooling water was circulated in the body part.

7

A water feed pipe, a pH modifier feed pipe, an acrylonitrile feed pipe, and a microbial cell catalyst feed pipe were each directly connected to the first reactor. In the first reactor, a reaction solution feed line equipped with a reaction solution feed pump was installed, and the reaction solution feed line was connected to the second reactor.

To the first reactor, 400 kg of water was charged in advance. The wet microbial cell obtained by the above-described culture method was suspended in pure water. The resulting suspension was continuously fed at a rate of 11 kg/h while stirring the contents of the first reactor. In addition, acrylonitrile having a purity of 99.8% was continuously fed to the first reactor at a rate of 32 kg/h via the acrylonitrile feed pipe. Pure water was also continuously fed to the first reactor at a rate of 37 kg/h via a pure water feed pipe. Cooling water having a temperature of 5°C was circulated in the jacket heat exchanger of the first reactor and the body part of the second reactor so as to control the temperature such that the reaction solution was maintained at 20°C during reaction. As a pH modifier, a 0.1M aqueous NaOH solution was continuously fed to the first reactor via a pH modifier feed pipe with the feed rate being adjusted such that the reaction solution had a pH of from 7.5 to 8.5. The pH of the reaction solution was measured at an outlet of the first reactor by a glass electrode method.

The reaction solution was continuously extracted from the first reactor at a rate of 80 kg/h such that the liquid level of the reaction solution during the reaction was maintained at a height of 1 m from the bottom of the tank, and the extracted reaction solution was continuously fed to the second reactor to allow the reaction to further proceed in the second reaction. The residence time in the first reactor and that in the second reactor were set at 10 hours and 5 hours, respectively.

The analysis was performed under the below-described HPLC conditions at 200 hours after the start of the reaction, and it was found that the conversion rate into acrylamide at the first reactor outlet was 90%, and the acrylonitrile concentration at the second reactor outlet was 40 ppm by mass. In addition, it was found that the acrylamide concentration at the second reactor outlet was 53% by mass.

The analysis conditions were as follows.

- Acrylamide analysis conditions:

  High-performance liquid chromatography apparatus: LC-10A system (manufactured by Shimadzu Corporation) (UV detector wavelength: 250 nm, column temperature: 40°C)
  Separation column: SCR-101H (manufactured by Shimadzu Corporation)
  Eluent: a 0.05% (by volume) aqueous phosphoric acid solution

- Acrylonitrile analysis conditions:

  High-performance liquid chromatography apparatus: LC-10A System (manufactured by Shimadzu Corporation) (UV detector wavelength: 200 nm, column temperature: 40°C)
  Separation column: WAKOSIL-II 5C18HG (manufactured by FUJIFILM Wako Pure Chemical Corporation)
  Eluent: an aqueous solution containing 7% (by volume) acetonitrile, 0.1 mM acetic acid, and 0.2 mM sodium acetate

[Example 2]

The production of acrylamide was performed in the same manner as in Example 1, except that the second reactor was configured to have 9 tubes of 114.3 mm in outer diameter, 105.3 mm in inner diameter, and 5 m in tube length, in place of the 15 tubes of 89.1 mm in outer diameter, 80.7 mm in inner diameter, and 5 m in tube length. The residence time in the first reactor and that in the second reactor were set at 10 hours and 5 hours, respectively.

The analysis was performed under the above-described HPLC conditions at 200 hours after the start of the reaction, it was found that the conversion rate into acrylamide at the first reactor outlet was 90%, and the acrylonitrile concentration at the second reactor outlet was 15 ppm by mass.

[Example 3]

The production of acrylamide was performed in the same manner as in Example 1, except that the second reactor was configured to have 6 tubes of 139.8 mm in outer diameter, 130.8 mm in inner diameter, and 5 m in tube length, in place of the 15 tubes of 89.1 mm in outer diameter, 80.7 mm in inner diameter, and 5 m in tube length. The residence time in the first reactor and that in the second reactor were set at 10 hours and 5 hours, respectively.

The analysis was performed under the above-described HPLC conditions at 200 hours after the start of the reaction, and it was found that the conversion rate into acrylamide at the first reactor outlet was 90%, and the acrylonitrile concentration at the second reactor outlet was the detection limit or lower (10 ppm by mass or lower).

[Example 4]

The production of acrylamide was performed in the same manner as in Example 1, except that the second reactor was configured to have 4 tubes of 165.2 mm in outer diameter, 155.2 mm in inner diameter, and 5 m in tube length, in place of the 15 tubes of 89.1 mm in outer diameter, 80.7 mm in inner diameter, and 5 m in tube length. The residence time in the first reactor and that in the second reactor were set at 10 hours and 5 hours, respectively.

The analysis was performed under the above-described HPLC conditions at 200 hours after the start of the reaction, and it was found that the conversion rate into acrylamide at the first reactor outlet was 90%, and the acrylonitrile concentration at the second reactor outlet was the detection limit or lower (10 ppm by mass or lower).

[Example 5]

[Preparation of Microbial Cell Containing Nitrile Hydratase]

A microbial cell of clone No. 3 was obtained in accordance with the method described in Example 1 of JP-ANo. 2001-340091, and the microbial cell was cultured in the same manner as in Example 1 of JP-ANo. 2001-340091, thereby obtaining a wet microbial cell containing nitrile hydratase.

[Production of Acrylamide]

An SUS tank reactor (capacity: 1 m$^3$) having a jacket heat exchanger, equipped with a stirrer, and having a tank inner diameter of 1 m and a straight body-portion length of 1.5 m, and an SUS double-tubular reactor having a capacity of 0.4 m$^3$ were prepared as a first reactor and a second reactor, respectively. The second reactor was configured to have a tube of 165.2 mm in outer diameter, 155.2 mm in inner diameter, and 20 m in tube length as an inner tube, and an outer tube of 267.4 mm in inner diameter as a jacket. The second reactor was vertically set, and a reaction solution was fed from a lower part of the inner tube, and cooling water was circulated in the outer tube.

A water feed pipe, a pH modifier feed pipe, an acrylonitrile feed pipe, and a microbial cell catalyst feed pipe were each directly connected to the first reactor. In the first reactor, a reaction solution feed line equipped with a reaction solution feed pump was installed, and the reaction solution feed line was connected to the second reactor.

To the first reactor, 400 kg of water was charged in advance. The wet microbial cell obtained by the above-described culture method was suspended in pure water. The resulting suspension was continuously fed at a rate of 11 kg/h while stirring the contents of the first reactor. In addition, acrylonitrile having a purity of 99.8% was continuously fed to the first reactor at a rate of 32 kg/h via the acrylonitrile feed pipe. Pure water was also continuously fed to the first reactor at a rate of 37 kg/h via a pure water feed pipe. Cooling water having a temperature of 5°C was circulated in the jacket heat exchanger of the first reactor and the outer tube of the second reactor so as to control the temperature such that the reaction solution was maintained at 20°C during reaction. As a pH modifier, a 0.1M aqueous NaOH solution was continuously fed to the first reactor via a pH modifier feed pipe with the feed rate being adjusted such that the reaction solution had a pH of from 7.5 to 8.5. The pH of the reaction solution was measured at an outlet of the first reactor by a glass electrode method.

The reaction solution was continuously extracted from the first reactor at a rate of 80 kg/h such that the liquid level of the reaction solution during the reaction was maintained at a height of 1 m from the bottom of the tank, and the extracted reaction solution was continuously fed to the second reactor to allow the reaction to further proceed in the second reaction. The residence time in the first reactor and that in the second reactor were set at 10 hours and 5 hours, respectively.

The analysis was performed under the above-described HPLC conditions at 200 hours after the start of the reaction, and it was found that the conversion rate into acrylamide at the first reactor outlet was 90%, and the acrylonitrile concentration at the second reactor outlet was the detection limit or lower (10 ppm by mass or lower). In addition, it was found that the acrylamide concentration at the second reactor outlet was 53% by mass.

[Example 6]

The production of acrylamide was performed in the same manner as in Example 5, except that the second reactor was configured to have a tube of 139.8 mm in outer diameter, 130.8 mm in inner diameter, and 28 m in tube length as an inner tube, and an outer tube of 267.4 mm as a jacket, in place of the tube of 165.2 mm in outer diameter, 155.2 mm in inner diameter, and a tube length of 20 m as the an inner tube, and the outer tube of 267.4 mm as the jacket, respectively. The residence time in the first reactor and that in the second reactor were set at 10 hours and 5 hours, respectively.

The analysis was performed under the above-described HPLC conditions at 200 hours after the start of the reaction, and it was found that the conversion rate into acrylamide at the first reactor outlet was 90%, and the acrylonitrile concentration at the second reactor outlet was the detection limit or lower (10 ppm by mass or lower).

[Example 7]

The production of acrylamide was performed in the same manner as in Example 5, except that three double-tubular reactors connected in series, each of which was configured to have a tube of 89.1 mm in outer diameter, 80.7 mm in inner diameter, and 25 m in tube length as an inner tube, and an outer tube of 155.2 mm in inner diameter as a jacket, was used in place of the second reactor configured to have the tube of 165.2 mm in outer diameter, 155.2 mm in inner diameter, and 20 m in tube length as the inner tube, and the outer tube of 267.4 mm in inner diameter as the jacket. The residence time in the first reactor and that in the second reactor were set at 10 hours and 5 hours, respectively.
The analysis was performed under the above-described HPLC conditions at 200 hours after the start of the reaction, and it was found that the conversion rate into acrylamide at the first reactor outlet was 90%, and the acrylonitrile concentration at the second reactor outlet was 30 ppm by mass.

[Comparative Example 1]

The production of acrylamide was performed in the same manner as in Example 1, except that the second reactor was configured to have 209 tubes of 27.2 mm in outer diameter, 21.6 mm in inner diameter, and 5 m in tube length, in place of the 15 tubes of 89.1 mm in outer diameter, 80.7 mm in inner diameter, and 5 m in tube length. The residence time in the first reactor and that in the second reactor were set at 10 hours and 5 hours, respectively.
The analysis was performed under the above-described HPLC conditions at 200 hours after the start of the reaction, and it was found that the conversion rate into acrylamide at the first reactor outlet was 90%, and the acrylonitrile concentration at the second reactor outlet was 120 ppm by mass.
The results of Examples 1 to 7 and Comparative Example 1 are shown in Table 1.

Table 1

|  | Tube diameter (m) | Tube length (m) | Number of tubes | Flow rate (kg/h) | Residence time (h) | Re number | Acrylonitrile concentration (ppm by mass) |
|---|---|---|---|---|---|---|---|
| Example 1 | 0.0807 | 5 | 15 | 80 | 5 | 7.8 | 40 |
| Example 2 | 0.1053 | 5 | 9 | 80 | 5 | 10.2 | 15 |
| Example 3 | 0.1308 | 5 | 6 | 80 | 5 | 12.7 | <10 |
| Example 4 | 0.1552 | 5 | 4 | 80 | 5 | 15.0 | <10 |
| Example 5 | 0.1552 | 20 | 1 | 80 | 5 | 60.8 | <10 |
| Example 6 | 0.1308 | 28 | 1 | 80 | 5 | 72.1 | <10 |
| Example 7 | 0.0807 | 25×3 | 1 | 80 | 5 | 117 | 30 |
| Comparative Example 1 | 0.0216 | 5 | 209 | 80 | 5 | 2.1 | 120 |

From the results shown above, it was found that acrylonitrile remaining in a reaction solution can be reduced by controlling the Re number to be in a given range in the second reactor having a plug-flow region.
The disclosure of Japanese Patent Application No. 2018-62126 filed on March 28, 2018, is hereby incorporated by reference in its entirety.
All publications, patent applications, and technical standards mentioned in this specification are herein incorporated by reference to the same extent as if each individual publication, patent application, or technical standard was specifically and individually indicated to be incorporated by reference.

**Claims**

1. A method of producing an amide compound, the method comprising:

obtaining a reaction solution including an amide compound by bringing a microbial cell including nitrile hydratase, or a processed product of the microbial cell, into contact with a nitrile compound in an aqueous medium in a first reactor; and

causing the obtained reaction solution including an amide compound to react in a second reactor having a plug-flow region,

a Reynolds number in the second reactor being controlled to from 5 to 1,000.

2. The method of producing an amide compound according to claim 1, wherein the Reynolds number in the second reactor is 10 or higher.

3. The method of producing an amide compound according to claim 1 or 2, wherein the Reynolds number in the second reactor is 100 or lower.

4. The method of producing an amide compound according to any one of claims 1 to 3, wherein the second reactor comprises a tube reactor that has a tube diameter of 10 cm or more.

## INTERNATIONAL SEARCH REPORT

International application No.

PCT/JP2019/013039

### A. CLASSIFICATION OF SUBJECT MATTER
Int.Cl. C12P13/02(2006.01)i, C07C231/06(2006.01)i, C07C233/09(2006.01)i

According to International Patent Classification (IPC) or to both national classification and IPC

### B. FIELDS SEARCHED
Minimum documentation searched (classification system followed by classification symbols)
Int.Cl. C12P13/02, C07C231/06, C07C233/09

Documentation searched other than minimum documentation to the extent that such documents are included in the fields searched
| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS(STN)

### C. DOCUMENTS CONSIDERED TO BE RELEVANT

| Category* | Citation of document, with indication, where appropriate, of the relevant passages | Relevant to claim No. |
|---|---|---|
| Y | JP 2013-162746 A (MITSUI CHEMICALS, INC.) 22 August 2013, claims, examples & WO 2011/148867 A1 & TW 201211260 A | 1-4 |
| Y | JP 2001-340091 A (MITSUI CHEMICALS, INC.) 11 December 2001, claims, paragraph [0048] & US 2002/0160466 A1, claims, paragraph [0052] & WO 2001/073101 A1 & EP 1182260 A1 & AU 4276901 A & AU 767517 B & CN 1320705 A & KR 10-0549598 B1 & TW I296652 B | 1-4 |

☒ Further documents are listed in the continuation of Box C.    ☐ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 24.04.2019 | 28.05.2019 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2019/013039

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2015-120764 A (ASAHI GLASS CO., LTD.) 02 July 2015, claims 1, 6, 12, paragraphs [0024], [0025] & US 2012/0022286 A1, claims 1, 6, 12, paragraphs [0030], [0031] & WO 2010/117029 A1 & EP 2418199 A1 & KR 10-2012-0005454 A & CN 102369184 A | 1-4 |
| Y | JP 2010-241908 A (KANEKA CORPORATION) 28 October 2010, claims 1, 5, 6, paragraph [0029], example 1 (Family: none) | 1-4 |
| Y | JP 2000-7710 A (MITSUI CHEMICALS, INC.) 11 January 2000, claims, paragraph [0021] (Family: none) | 1-4 |

Form PCT/ISA/210 (continuation of second sheet) (January 2015)

**EP 3 778 911 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2003000914 A
- JP 2001340091 A
- JP 2013162746 A
- JP 2018062126 A